# EUROPEAN PATENT SPECIFICATION

(11) **EP 0 785 433 B1**
(45) Date of publication and mention of the grant of the patent: **26.11.2003**
(21) Application number: 96303456.6
(22) Date of filing: 15.05.1996
(51) Int. Cl.: G01N 35/00, C12M 1/34, B01L 3/00, B29C 45/00

(54) **Test sample card**
Testkarte für Analyser
Carte test pour analyses

(30) Priority: 17.01.1996 US 587633
(43) Date of publication of application: 23.07.1997
(60) Divisional application: 01203837.8
(73) Proprietor: bioMerieux Vitek, Inc., Hazelwood, Missouri 63042 (US)
(72) Inventor: O'Bear, Raymond E., Granite City, Illinois 62040 (US); Staples, John, Florissant, Missouri 63031 (US); Tegeler, Garry R., Hazelwood, Missouri 63042 (US)
(74) Representative: Froud, Clive

(56) References cited:
- EP-A- 0 282 840
- EP-A- 0 526 222
- EP-A- 0 703 052
- EP-A- 0 745 856
- WO-A-94/18369
- FR-A- 2 350 593
- FR-A- 2 368 774
- US-A- 3 880 978
- US-A- 4 074 940
- US-A- 4 318 994
- GLENN BEALL: "plastic part design part 3" DESIGN ENGINEERING., September 1985, LONDON GB, pages 75-80, XP002036568

## Description

This invention relates to a test sample card; more particularly, it relates to the field of test sample cards and similar devices that hold samples for analysis by an optical system. Such sample cards are typically employed in chemical or biological sample testing systems.

Test sample cards typically have a plurality of small sample growth or reaction wells that are arranged in various arrays. The cards also have a fluid passage network that connects the growth wells to a fluid intake port. During manufacture of the card, one side of the card is taped with a clear adhesive tape to seal off one side of the wells. The individual wells are then loaded with a small quantity of chemicals or reagents, such as various growth media for bacteria, or various concentrations of different antibiotics or other drugs. After the growth wells are loaded with chemicals, the other side of the card is sealed by a clear adhesive tape, sealing the other side of the wells.

When the cards are to be put in use, the wells of the card are loaded with the sample, for example a fluid containing a biological sample from a patient. The loading of the wells may be achieved by inserting one end of a straw-like transfer tube into the fluid intake port, and placing the other end of the transfer tube into a test tube containing the sample, thereby placing the fluid intake port in fluid communication with the sample. The test tube and card/transfer tube assembly is then placed in a vacuum chamber. Vacuum is applied to the chamber and then the chamber is vented to atmosphere. The venting process causes the fluid in the test tube to enter the intake port and travel along the fluid passage network to the growth wells.

Typically, the cards are provided with a bubble trap connected to the sample well. The user orients the card such that the bubble trap is positioned above the sample well and then gives the card a light tap, causing any air bubbles in the well to move into the bubble trap.

In a microbiological testing application for the card, after the card is loaded with a sample the card is incubated for a period of time, and then read by an optical system. The optical system typically employs some form of transmittance light source that illuminates the wells of the card, and a detector arrangement that measures the transmittance of light through the wells. The amount of transmittance depends on the reaction between the sample and the growth media or drugs placed in the growth wells. The transmittance measurements for the wells of the card permits an identification of an unknown agent in the sample, or the susceptibility of the agent to different antibiotics or other drugs, or the detection of a test reaction product.

Test sample cards of the prior art include a 30-well sample card which is described in U.S. Patent No. 4,318,994 to Meyer et al. Other patents relating to the general subject of test sample cards include the Aldridge et al. patent, U.S. No. 3,963,355; the Fadler et al. patent, U.S. No. 4,038,151; the Robinson et al. patent, U.S. No. 5,374,395; and the Charles et al. patents, U.S. Nos. 4,188,280 and 4,116,775. The Charles et al. patents also describe a card reading system for the 30-well cards described in the Meyer et al. patent.

As microbiological science has advanced over the past few decades, scientists have been able to isolate greater numbers of bacteriological agents and design suitable growth media and antibiotics for these agents. This development has resulted in a need in the art for test sample cards that have a greater number - of growth wells. This need applies to both identification and susceptibility types of cards. Ideally, such a redesigned card would have the same physical dimensions and external features as the earlier generation of cards, so as to permit the redesigned card to be read by existing optical reading machines.

Placing greater numbers of wells on a card with fixed dimensions is not a simple matter of adding more wells to the cards. Rather, adding more wells to the card has the potential of increasing the possibility of inter-well cross contamination, a phenomenon known in the art as "cross-talk". Cross contamination of samples or reagents between adjacent wells can give erroneous test results when the cards are read. For example, by simply adding more wells to the 30 well card described in the above Meyer et al. patent, inter-well contamination can result. To understand the difficulty in achieving higher well counts in a card of fixed dimensions, the key issue of cross-contamination between wells and how that can affect the performance of test sample cards will be discussed in further detail.

By placing more wells on a given amount of space on the card, the wells are placed closer together. Since all the wells are indirectly in fluid communication with each other by the card's fluid channel network, cross-contamination can result from sample, growth media or reagents diffusing along the fluid channel network from one well to an adjacent well, given enough time. Some types of cards may require incubation times of up to 18 hours, which is enough time for cross-contamination to occur if the wells are too close together. Thus, increasing the number of wells in the card poses a challenge in avoiding this type of cross contamination.

In the present invention, the inventors have solved this problem by designing a fluid channel passage network that achieves a sufficient separation distance between adjacent wells (as measured along interconnecting fluid channels), while also achieving an increased areal density of wells in the card.

The inventors have also discovered that the cross-talk problem is to some extent determined by how the molten plastic flows in the card mold during the manufacture of the card. The inventors have discovered that cross-contamination can occur by virtue of the sample media travelling along very tiny fissures or cracks that can form in the surface of the cards. These cracks, known as "knit lines", are inevitably created when two flow paths of molten plastic material meet during the card molding process. The inventors have appreciated that cross-talk can be minimized by controlling the formation in the knit lines in such a manner that they are reduced in number, and that they are oriented in a direction that is least likely to result in knit lines bridging adjacent wells.

Thus, the present invention provides design features in a test sample card that substantially reduces, if not eliminates, the probability of inter-well contamination. These features include the above-described special fluid channel networks. The invention further provides new coring techniques to improve the flow of card material during manufacture and the consequent control or elimination of knit lines that can cause inter-well contamination. Thus, the invention achieves the unexpected result of obtaining a larger number of wells per given area than prior art cards, while actually reducing the risk of cross-contamination.

The inventive test sample card possesses additional advantages and improvements. As noted above, a major issue in the design of sample cards is how the card handles the situation where air bubbles are present in the growth wells. The presence of air bubbles may be due to less than perfect vacuum conditions when the cards are loaded with samples, or as a byproduct of chemical reactions and biological processes taking place when the card is incubated with samples in the wells. The air bubbles have a tendency to distort the transmittance measurements. The invention provides for improved bubble trap and sample well features that facilitate the removal of air bubbles from the sample well into the bubble trap, and preventing of the air bubble, once in the bubble trap, from reentering the growth well. These features substantially improve the reliability of the transmittance measurements.

In one embodiment, the present invention provides a test sample card comprising a body made from a molded material, the said body comprising a fluid entrance port and first and second end regions and first and second side regions, the said body defining a plurality of wells placed between the said first and second end regions and the said first and second side regions, the said body further comprising a fluid passage network connecting the said fluid entrance port to the said wells, characterised in that the said body has cored regions disposed in at least one of the said first and second end regions or the said first and second side regions, which cored regions improve the flow of the said molded material during the manufacture of the said card and inhibit the formation of knit lines in the said material so as to reduce the likelihood of contamination between different wells in the said card.

Preferably, in such a card, the said wells are arranged in a series of rows in the said card with five wells per row, and the said fluid channels between the said port and the said five wells of the said row are disposed in the said top and bottom surfaces of the said card such that two fluid channels are disposed on one of the said surfaces of the said card leading to two of the said wells in the said row of wells and three fluid channels are disposed on the other of the said surfaces to the remaining three of the said wells in the said row of wells.

In a further embodiment, the present invention provides a mold for manufacturing a test sample card from a card material characterised in that it comprises a plurality of sample wells, the said card having an exterior surface after ejection of the said card from the said mold, the said mold. having at least one positive element creating at least one cored section in the said card adjacent to the said wells, the said positive element directing the flow of the said card material in the said mold so as to control the formation of knit lines in the surface of the said card substantially to prevent the said knit lines from forming in a predetermined direction.

Having indicated the scope of the present invention, it will now be further described and illustrated in more general terms.

A test sample card is provided having a body made from a molded material, the body comprising a fluid entrance port and first and second end regions and first and second side regions. A plurality of growth or reaction wells are located in the card body between the first and second end regions and the first and second side regions. A fluid channel network connects the fluid entrance port to said growth wells. To improve the flow of the material during the molding process, cored regions are disposed in at least one of the first and second end regions or the first and second side regions. The cored regions improve the flow of the molded material during the manufacture of the card and inhibit or control the formation of knit lines in the surface of the card in a manner so as to reduce the likelihood of contamination between different wells in the card.

In a preferred embodiment, the cored region is disposed along one of the sides of the card below the identification area on the card, and includes a plurality of dam features directed towards the growth wells. The dam features help prevent the formation of knit lines in the row direction (i.e., the direction defined by the wells in a single row), which is the direction having the shortest separation distance between adjacent rows as measured along the surface of the card. Cored features may also be formed at the ends of the card and along both sides, again helping prevent the formation of knit lines in the surface of the card and controlling the knit lines such that they form in a manner unlikely to bridge adjacent wells.

In another aspect of the invention, the card has a fluid channel network linking the fluid intake port to the wells, comprising a first set of fluid channels disposed in the top surface of the card and a second set of fluid channels disposed in the bottom surface of the card. The first and second fluid channels supply fluid to multiple wells in every row of wells in the card. The fluid channels are arranged on the card so as to increase the separation distance between adjacent wells, as measured along the fluid channels, thereby reducing the risk of contamination between adjacent wells. In a preferred embodiment, the separation distance is greater than or equal to 2.5 cms (1 inch) in a card measuring roughly 8.9 cms by 5.7 cms (3 ½ inches by 2 ¼ inches).

In yet another aspect of the invention, at least one of the wells has a bubble trap in communication with the well via a bubble trap passage. The bubble trap passage has a well end and a bubble trap end, wherein the width of the well end of the bubble trap passage is greater than the width of the bubble trap end of the bubble trap passage. This funnel-shaped bubble trap passage promotes the collection of air bubbles in the bubble trap. At the intersection of the walls forming the bubble trap passage and the bubble trap, a restriction is formed in the bubble trap passage. The restriction, preferably in the shape of a sharp comer, prevents any air bubbles in the bubble trap from migrating from the bubble trap back into the growth well.

A further embodiment of the invention provides for raised rail features along the sides of the card, which facilitate the stacking of cards without scuffing of the adhesive tape covering the cards. The scuffing can interfere with the optical measurements, and if serious enough, could disturb the adhesive tape and increase the possibility of inter-well contamination.

An object of the invention is to provide a test sample card with a reduced probability of cross-talk from adjacent growth wells.

A further object of the invention is to provide a card molding features which control knit lines and prevent them from forming in a manner that could contribute to cross-talk.

The present test sample card may also have a bubble trap passage that facilitates.the collection of bubbles in a bubble trap, and prevents the bubbles from leaving the bubble trap and reentering the growth wells.

Presently preferred embodiments of the invention are depicted in the drawings, wherein like reference numerals refer to like elements in the various views, and wherein:
Figure 1 is a plan view of the bottom side of the card;
Figure 2 is a plan view of the top side of the card;
Figure 3 is a side view of the card;
Figure 4 is a side view of the card opposite to the side of Figure 3;
Figure 5 is an end view of the card;
Figure 6 is an end view showing the opposite end of Figure 5;
Figure 7 is a detailed plan view of the wells of the card that receive fluid samples from the through-card feed channels of Figure 2;
Figure 8 is a detailed plan view of the wells of the card that receive sample fluid from the feed channels in the bottom surface of the card;
Figure 9 is a sectional view of the wells along the lines 9-9 of Figures 7 and 8;
Figure 10 is a sectional view of the bubble trap along the lines 10-10 of Figures 7 and 8;
Figure 11 is a sectional view along lines 11-11 of the through-card feed channel of Figure 7;
Figure 12 is an enlarged plan view of the bubble trap passage showing the relationship between the dimensions of the entrance and restriction portions of the bubble trap passage; and
Figure 13 is a plan view of a portion of the card shown greatly enlarged in order to illustrate how the cored sections control the formation of knit lines int he surface of the card.

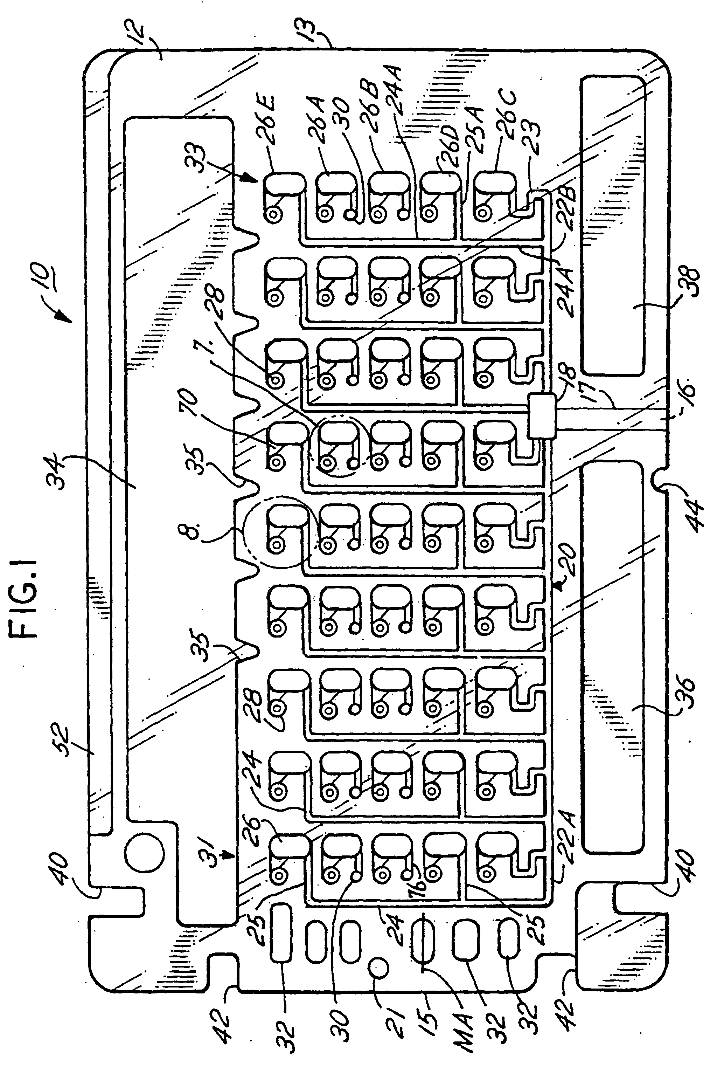

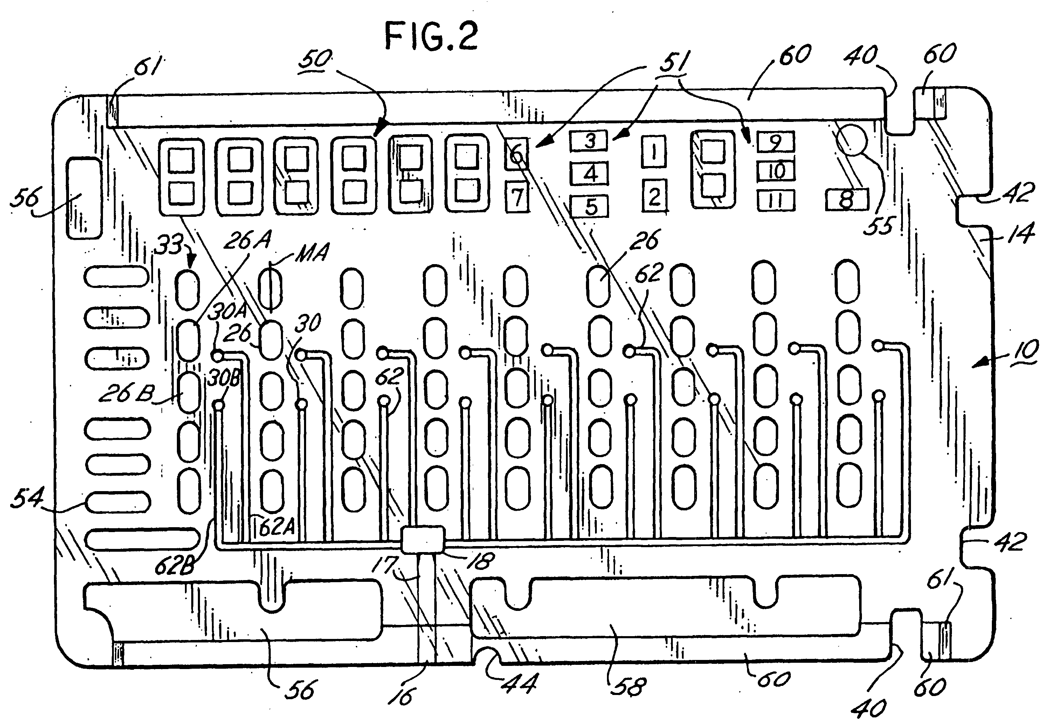

Figure 1 shows the bottom surface 12 of a preferred test sample card 10 in accordance with a preferred embodiment of the invention. The surface 12 is referred to as the "bottom" due to the fact that it receives clear adhesive tape first, and is placed face down during the loading of the wells with growth media or other chemicals or reagents. The card 10 has a rear edge 13, a front edge 15 and a top surface 14 shown in a plan view in Figure 2. Preferably, the card 10 is manufactured using an injection molding process from crystal polystyrene, such as Dow Chemical Company Styron™ 666D crystal polystyrene, or the equivalent.

Referring to Figures 1, 2 and 4, a fluid intake port 16 is located on the side of the card 10 which provides an opening for fluid containing biological or control or chemical samples to enter into the interior of the card 10. The port 16 is connected to a fluid intake passage 17 that leads to an intake manifold 18.

A fluid channel network 20 comprising a pattern of full-radius, shallow (e.g., 0.03 cms (0.012 inches) deep) grooves, is provided on the bottom surface 12. During the card loading process, fluid travels from the intake manifold 18 along the network 20 to a plurality of growth wells 26 arranged in an array in the card 10. The fluid passage network 20 includes primary fluid channels 22A and 22B extending horizontally to the left and right, respectively, of the manifold 18; a set of secondary fluid channels 24 extending upwardly from the fluid channels 22A and 22B; and a set of ternary fluid channels 25 which lead from the secondary channels 24 to the growth wells 26.

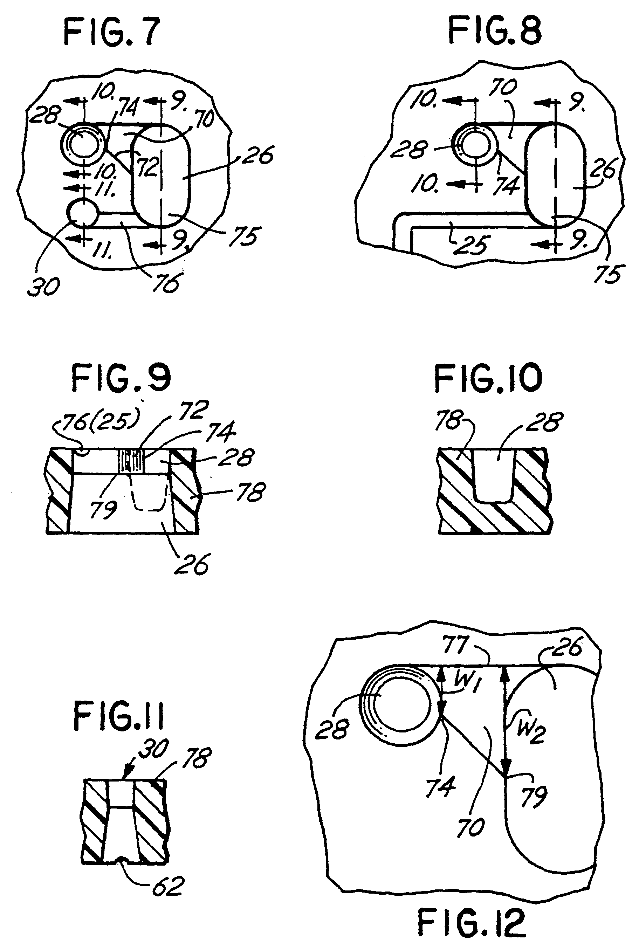

Each of the growth wells 26 contains a bubble trap 28 which is in communication with the growth wells 26 by a bubble trap passage 70. The bubble traps 28 and bubble trap passages 70 are discussed in detail in conjunction with Figures 7-12 below.

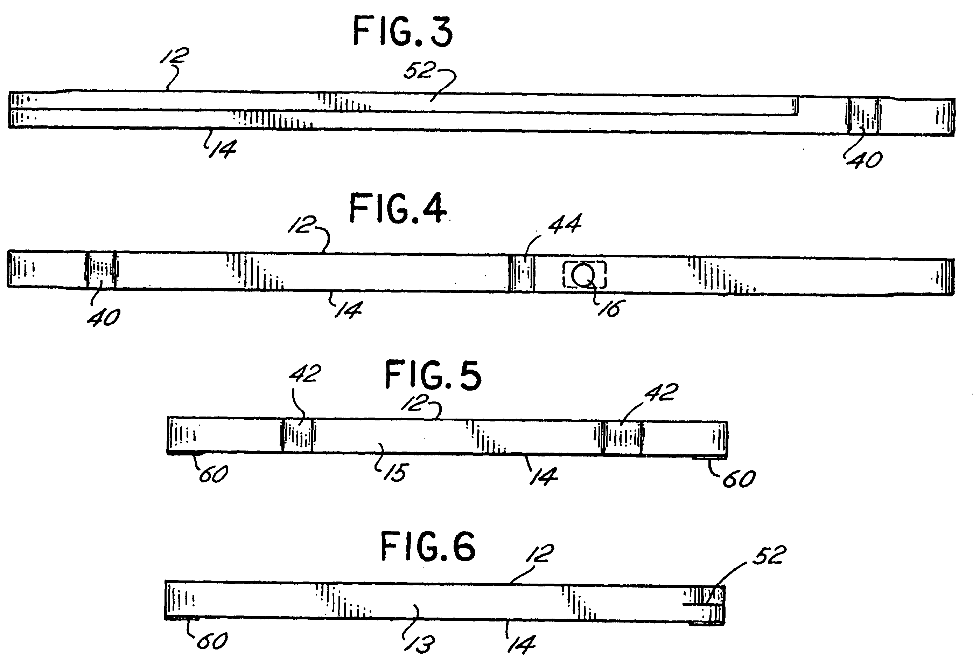

As shown in Figures 1 and 6, the card 10 has a pair of opposed gripping slots 40 at the front end 15 of the card, which are gripped by mechanical structures in the card reader (not shown) for the card 10. Other gripping features could be provided for other types of readers. A pair of positioning notches 42 are provided in the front edge 15 of the card, which assist in the placement of the card 10 in proper alignment with the optical system for the card reader. A tray keeper notch 44 is provided along the side of the card 10 adjacent to the port 16. As shown in Figures 2, 3, and 6, an alignment groove 52 is provided on the bottom surface of the card to ensure that the user correctly inserts the card in the reading machine. Alternative insertion and orientation features could of course be provided. The top surface of the card 10 is shown in Figure 2, and includes a second set

of fill channel grooves 62 in communication with the manifold 18. The fill channels 62 each terminate in a through-card feed channel 30, which passes through the card 10 from the top side of the card shown in Figure 2 to the bottom side of the card 10 shown in Figure 1. The through-card feed channel 30 conducts fluid samples to a fill channel 76, shown in Figures 1 and 7. The fill channels 76 conduct sample fluid into the growth wells 26. Referring now to Figure 7, the growth wells 26 that receive fluid samples

from the through-card feed channels 30 are shown in greater detail. The through-card feed channel 30 is shown in a vertical cross-sectional view in Figure 11. The fluid sample passes through the card 10 from the top side of the card (Figure 2) to the bottom side of the card (Figure 1) via the through-card feed channel 30, and then enters a fill channel 76. The fill channel 76 leads to the bottom portion 75 of the well 26.

From Figures 1 and 2, it can be seen that fluid channels are provided on both sides of the card 10. In particular, from Figures 1 and 2 it can be seen that the fluid channels on both sides of the card 10 provide an inter-well separation distance of at least 2.5 cms (1 inch) in a card with dimensions of approximately 8.9 cms by 5.7 cms (3 ½ inches by 2 ¼ inches), with the inter-well separation distance measured along the fluid channels that connected the wells together. For example, referring to the left hand side of the top surface of the card (Figure 2), fluid channel 62A provides a pathway for sample fluids to the growth well 26A via the through-card feed channel 30A. Well 26A is in communication with well 26B via channel 30A, fluid passages 62A and 62B, and channel 30B. By virtue of the elongate fluid channels 62A and 62B, the inter-well separation distance of wells 26A and 26B is on the order of 3.8 cms (1 ½ inches) in the illustrated embodiment. The separation distance is sufficiently great that the probability of inter-well contamination along the passages 62A and 62B is exceedingly remote, even with a long incubation time for the card. For most microbiological applications, in which the sample remains in the wells for some time during incubation and reading, a minimum desirable separation distance as measured along the fluid channels is at least 1.9 cms (0.75 inches), with at least 2.5 cms (1 inch) preferred. The greater the separation distance, the less likely that diffusion of fluids or reagents between wells along the fill channels will occur.

Referring to the right-hand side of Figure 1, a potential cross-contamination pathway exists between wells 26C and 26D via fluid channels 23, 22B, 24A and 25A. However, the channels, 23, 22B 24A and 25A are designed such that the distance from well 26C to well 26D is greater than 2.5 cms (1 inch), substantially reducing the possibility of inter-well contamination. The channel 23 is given an "S" shape to increase the distance from the channel 22B to the well 26C. Note further that well 26D has a potential cross-contamination pathway with well 26E via fluid channels 25A and 24A but this distance again is at least 2.5 cms (1 inch) in the illustrated embodiment, substantially eliminating the likelihood of inter-well contamination between wells 26E and 26D.

As can be seen from Figures 1 and 2, the wells 26 in the card are arranged in an array of multiple rows of wells 26, each of the rows of wells having a common fluid channel network described for the row 33 adjacent to the end 13 of the card.

As shown in Figure 2, an identification area 50 is provided on the surface 14 of the card 10. The area 50 includes a series of "8"s which provide an area for a technician to write identification numbers associating the test sample card 10 with the particular patient or test. A card code block 51 is placed adjacent the area 50 and includes eleven identification blocks identifying the type of card depending on how the blocks are marked. An external test result (ETR) mark location 55 is also provided on the top surface.

Referring to Figure 1, the card 10 further includes a first row 31 adjacent to end 15. A set of oval-shaped cored pockets 32 are provided having a major axis MA (Figure 1) oriented towards the adjacent row 31 of wells 26. The molten plastic card material enters the mold at the molding gate region 21. The cored pockets 32 enhance the flow of the material during the molding of the cards 10. Specifically, the mold for the cards has positive elements that create the cored pockets 32 to help prevent the formation of knit lines adjacent to the first row 31 of wells 26 and prevent the formation of knit lines in the direction along the major axis MA (Fig. 2) of the wells 26 (i.e., the row direction where the separation distance between the wells measured along the surface of the card is the least). Referring to Figure 2, a set of oval cored pockets 54 are also provided to reduce the possibility of knit lines being formed adjacent to the last row 33 of wells 26 in a manner parallel to the major axis of the wells. A preferred depth of the cored pockets 32 and 54 (height of the positive elements) is 0.18 cms (0.07 inches), but this depth is not especially critical.

While oval shaped cored pockets are illustrated, other shapes could be used. Preferably, the shapes are either oblong or elongate with the long axis of the pocket oriented towards the adjacent row of wells, as shown in the example of oval pockets in Figures 1 and 2. This promotes a flow of molten plastic and control of knit lines such that the knit lines form, if at all, in a manner roughly parallel to the long axis of the pocket: the direction in which the inter-well separation distance as measured along the surface of the card is the greatest.

The side of the card 10 adjacent to the primary fluid channel 22A and 22B also contains cored sections 36 and 38. These cored sections control the formation of knit lines on the surface of the card 10 during the molding of the card, particularly in the vicinity adjacent to the first column of wells immediately adjacent to the primary fluid channels 22A and 22B.

Another cored section 34 is provided on the opposite side of the card as shown in Figure 1, adjacent to the wells 26 in the far column of wells. The cored section 34 is preferably between 0.051 and 0.127 cms (between 0.020 and 0.050 inches), so as to not interfere with the reading of the identification marks on the top surface of the card. A preferred depth of the cored section 34 is 0.076 cms (0.030 inches). The cored section 34 is located on the bottom side of the card as shown in Figure 1 directly behind the identification area 50, shown in Figure 2. With a depth of 0.076 cms (0.030 inches), the reading of the identification information in the regions 50, 51, 55 by an optical system in the card reader is not adversely affected.

The cored section 34 improves the flow of molten plastic material over the card during the manufacture of the card, and controls the formation of knit lines in the surface of the card. As shown in Figure 1, the cored section 34 preferably includes a set of V-shaped dam portions 35 which improve the flow of the material during the molding of the card 10. The dam portions 35 (comprising extensions of the cored section 24 with a recess of, for example 0.076 cms (0.03 inches)) prevent knit lines from forming in a direction parallel to the major axis of the wells 26, where the separation distance (as measured along the surface of the card) is the least. Instead, the knit lines form, if at all, generally parallel to the minor axis of the wells, and thus are unlikely to bridge two wells.

In accordance with the above, persons of skill in the art of plastic molding will appreciate that the mold for manufacturing the test sample card has positive elements that create the recessed cored sections 32, 34, 54, 60 and the dam feature 35. For the cored sections 32, the positive elements comprise a set of an elongate oval elements positioned adjacent to the elements that define the rows of wells. The elongate elements have a major or long axis MA oriented perpendicular to the row direction. The positive elements direct the flow of card material in the mold during molding in a manner so as to control the formation of knit lines in the surface of the card.

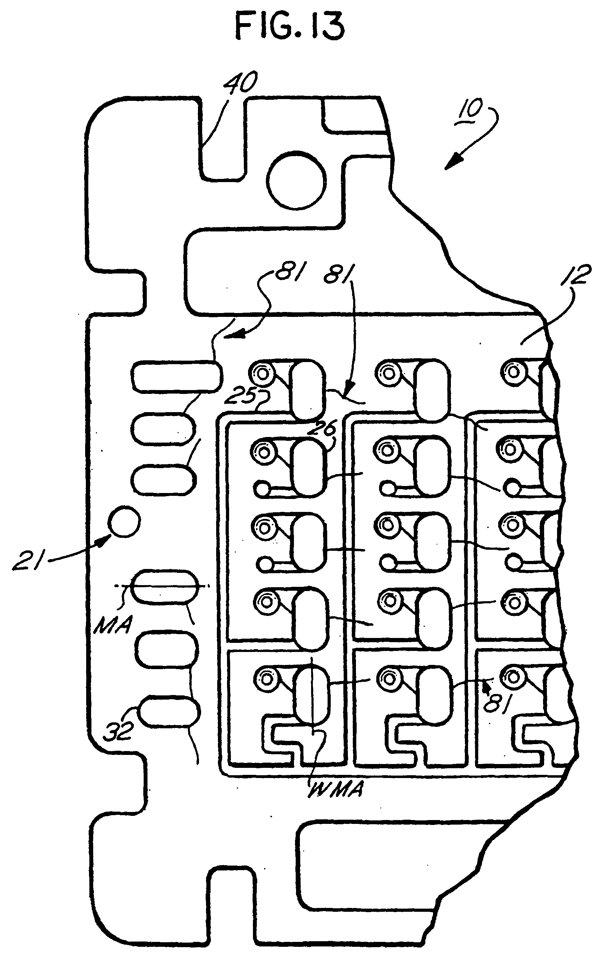

Referring to Figure 13, a portion of the bottom side 12 of the card 10 is shown enlarged in order to illustrate the how the cored sections control the formation of knit lines. The card material enters the mold at the gate 21. The knit lines 81 are tiny fissures (also exaggerated in thickness in Figure 13) that form when plastic flows from the gate around the positive elements that form the cored sections 32. The flow of plastic in the mold is such that the knit lines 81 in the surface 12 of the card tend to form in a direction generally perpendicular to the well major axis MWA, which is the direction in which the inter-well separation distance (measured along the surface of the card) is the least. The same effect is achieved by the other cored sections in the card, including the dam features 35 in the cored section 34.

Referring to Figures 7 and 9, the card portion 78 surrounding the well 26 includes a slanted wall portion 72 that cooperates with wall 77 to form a generally funnel-shaped region in the bubble trap passage 70. The funnel-shape bubble trap passage 70 includes a restriction 74 comprising a sharp comer at the intersection of the bubble trap passage 70 and the bubble trap 28. In use, when the sample fluid enters the card and fills the well 26, the technician holds the card 10 in a position such that the bubble traps 28 are generally in an upward or vertically inclined position relative to the wells 26. The technician jiggles the card with a tap or a snap of the wrist, jiggling any air bubbles that may be present within the well 26 into the bubble trap 28. Taking advantage of the surface tension and meniscus properties of the sample fluid, the sharp comer restriction 74 serves to prevent any air bubbles that may be present in the bubble trap 28 from re-entering the well 26.

The geometry of the bubble trap passage 70 is shown schematically in Figure 12. At the intersection of the bubble trap passage 70 and the bubble trap 28, the width between the restriction 74 and the wall 77, W1, is less than 0.1 cms (0.04 inches), with 0.08 cms (0.033) inches in a preferred embodiment. At the entrance of the bubble trap passage, the width between the comer 79 and the wall 77, W2, is greater than W1 and 0.17 cms (0.066 inches) in the preferred embodiment. The relatively wide distance W2 allows air bubbles in the well 26 to be slinged into the bubble trap passage 70 and the bubble trap 28 when the card is tapped by the user. The narrow restriction at W1, and especially the sharp restriction corner 74, prevents the air bubble from migrating out of the bubble trap 28 and back into the well 26.

Referring to Figure 8, the construction of the wells that receive fluid samples along the fluid channels 25 is the same as that described in Figures 7 and 9. Referring to Figure 10, the bubble trap 28 is shown in a sectional view along the lines 10-10 of Figures 7 and 8.

An additional feature to the card 28 is a rail feature that is used to separate cards from each other when the cards are stacked. This rail feature is especially useful when the cards are ejected from the mold and stacked on top of each other. The separation of the cards prevents any scuffing of the surface of the cards. After tape has been applied to the cards, the rails also prevent possible damage to the adhesive tape applied to the cards. Referring to Figures 2, 5 and 6, a pair of raised rail features 60 are provided on opposite edges of the card on one side of the card. The raised rail features 60 extend along substantially the entire length of the card 10. The height of the raised rails 60 need only be enough to prevent the adhesive tape covering the wells from touching when the cards are stacked. A height of 0.015 cms (0.006 inches) or thereabouts for the rail features 60 is sufficient for the illustrated embodiment. The ramp features 61 facilitate the stacking operation by allowing the rails to slide over each other as the cards are slid into a stacked condition.

Preferred sample loading and reading machines for the card are available from bioMérieux Vitek, Inc., 595 Anglum Drive, Hazelwood, Missouri.

From the forgoing, it will be appreciated that some modification may be made from the preferred embodiment without departure from the scope of the invention. For example, the location of the cored sections in the card may be modified somewhat depending on the particular material chosen from the card, the location of wells, and the dimensions of the card. Further, the particular fluid channel design in the top and bottom surfaces of the card may be varied. For example, the channels could be designed in an alternating pattern, such that every other well in a row of wells receives fluid samples from fluid channels in the top and bottom surfaces of the card, thereby achieving an adequate inter-well separation distance.

## Claims

1. A test sample card (10) comprising a body made from a molded material, the said body comprising a fluid entrance port (16) and first and second end regions (13, 15) and first and second side regions, the said body defining a plurality of wells (26) placed between the said first and second end regions and the said first and second side regions, the said body further comprising a fluid passage network (20) connecting the said fluid entrance port to the said wells, **characterised in that** the said body has cored regions (32, 34, 54, 60) disposed in at least one of the said first and second end regions or the said first and second side regions, which cored regions improve the flow of the said molded material during the manufacture of the said card and inhibit the formation of knit lines (81) in the said material so as to reduce the likelihood of contamination between different wells in the said card.

2. A card as claimed in claim 1 wherein the said cored regions are disposed in at least one of the said first and second end regions of the said card, the said wells are arranged in a plurality of parallel rows having a first row adjacent to the said first end region and a last row adjacent to the said second end region, and the said cored regions comprise a first set of cored pockets oriented across the said first end region substantially parallel and adjacent to the said first row and a second set of cored pockets oriented substantially parallel and adjacent to the said last row, preferably the said first and second core pockets comprising elongate oval coring pockets defining a major axis arranged such that the major axis is oriented perpendicular to the said first and last rows.

3. A card as claimed in claim 1 or claim 2 wherein the said cored regions are disposed in at least one of the said first and second side regions and the said cored regions comprise at least one dam portion (35) disposed at the boundary of the said cored region and the non-cored portion of the said card.

4. A card as claimed in any of claims 1 to 3 wherein the said card has a top surface (14) and an identification region (50) in the said top surface, and a bottom surface (12) and a cored region in the said bottom surface below the said identification region, preferably the said card being made from injection molded crystal polystyrene.

5. A card as claimed in any of claims 1 to 4 wherein the said cored region has a depth of between 0.051 and 0.127 cms (between 0.02 and 0.05 inches), preferably substantially 0.076 cms (0.03 inches).

6. A card as claimed in claim 1 comprising a body comprising a fluid entrance port and first and second end regions and first and second side regions, the said body defining a plurality of wells placed between the said first and second end regions and the said first and second side regions, the said body further comprising a fluid passage network connecting the said fluid entrance port to the said wells, **characterised in that** at least one of the said wells has a bubble trap (28) in communication with the said well via a bubble trap passage (70), the said bubble trap passage having a well end and a bubble trap end, the width of the said well end being greater than the width of the said bubble trap end, and the intersection of the said bubble trap end of the said bubble trap passage and the said bubble trap forming a restriction (74) in the said bubble trap passage, the said bubble trap passage facilitating the collection of air bubbles which may be present in the said wells in the said bubble trap and the said restriction preventing air bubbles from migrating from the said bubble trap into the said well.

7. A card as claimed in claim 6 wherein the said well comprises a first end portion and an opposite second end portion, the said bubble trap being located adjacent to the said first end portion and a fluid entrance passage entering the said well at the said second end portion.

8. A card as claimed in claim 6 or claim 7 wherein the said restriction comprises a sharp comer at the intersection of the said bubble trap passage and the said bubble trap, the said sharp comer preventing bubbles from moving from the said bubble trap into the said bubble trap passage and into the said well.

9. A card as claimed in any of claims 6 to 8 wherein the said fluid passage network connects the said fluid entrance port with the said well by fluid channels disposed on the front and rear surfaces of the said card thereby to increase the separation distance between adjacent wells.

10. A card as claimed in claim 1 wherein it comprises a body comprising a fluid entrance port and first and second end regions and first and second side regions, the said body defining a plurality of growth wells placed between the said first and second end regions and the said first and second side regions, the said body further comprising a top surface and a bottom surface and a fluid passage network connecting the said fluid entrance port to the said growth wells, **characterised in that** the said fluid passage network comprises a first set of fluid channels disposed in the said top surface of the said card and a second set of fluid channels disposed in the said bottom surface of the said card, the said first and second sets of fluid channels being arranged on the said card so as to provide an adequate separation distance between adjacent wells as measured along the said fluid channels thereby reducing the risk of contamination between adjacent wells.

11. A card as claimed in claim 10 wherein the said separation distance is greater than 1.9 cms (0.75 inches), preferably greater than 2.5 cms (1 inch).

12. A card as claimed in claim 10 or claim 11 wherein the said wells are arranged in a series of rows in the said card with five wells per row, and the said fluid channels between the said port and the said five wells of the said row are disposed in the said top and bottom surfaces of the said card such that two fluid channels are disposed on one of the said surfaces of the said card leading to two of the said wells in the said row of wells and three fluid channels are disposed on the other of the said surfaces to the remaining three of the said wells in the said row of wells.

13. A card as claimed in claim 1 wherein it comprises a body having a top surface and a bottom surface, adhesive tape being applied to the said top and bottom surfaces, a fluid entrance port and first and second end regions and first and second side regions, the said body defining a plurality of wells placed between the said first and second end regions and the said first and second side regions, the said body further comprising a fluid passage network connecting the said fluid entrance port to the said wells, **characterised in that** it comprises a pair of rails (60) upwardly extending from one of the said top or bottom surfaces, the said rails permitting the said cards to be stacked without touching of adhesive tape applied to adjacent cards in the said stack of cards.

14. A card as claimed in claim 13 wherein the said rails are oriented along the said first and second side regions between the said ends of the said card.

15. A card as claimed in claim 6 wherein the said restriction in the said bubble trap passage has a width, W1, of less than 0.1 cms (0.04 inches).

16. A mold for manufacturing a test sample card from a card material **characterised in that** it comprises a plurality of sample wells, the said card having an exterior surface after ejection of the said card from the said mold, the said mold having at least one positive element creating at least one cored section in the said card adjacent to the said wells, the said positive element directing the flow of the said card material in the said mold so as to control the formation of knit lines in the surface of the said card substantially to prevent the said knit lines from forming in a predetermined direction.

17. A mold as claimed in claim 16 wherein the said wells are arranged in a plurality of rows defining a row direction and the said positive element comprises an elongate element positioned adjacent to the said rows and having a long axis oriented perpendicular to the said row direction, preferably the said positive element comprising a dam feature located adjacent to the said wells.

## Patentansprüche

1. Eine Probenanalysekarte (10), welche einen Grundkörper aufweist, welcher aus einem geformten bzw. gepressten Material besteht, wobei dieser Grundkörper aufweist: einen Flüssigkeitseingangsanschluss (16) und erste und zweite Endbereiche (13, 15) und erste und zweite Seitenbereiche, wobei der Grundkörper eine Vielzahl von Wannen (26) definiert, welche zwischen den ersten und zweiten Endbereichen und den ersten und zweiten Seitenbereichen platziert sind, wobei der Grundkörper ferner ein Flüssigkeitsdurchlaufnetzwerk (20) aufweist, welche den Flüssigkeitseingangsanschluss mit den Wannen verbindet, **dadurch gekennzeichnet, dass** der Grundkörper hohle Bereiche (32, 34, 54, 60) besitzt, welche wenigstens in einem der ersten und zweiten Endbereiche oder den ersten und zweiten Seitenbereichen angeordnet sind, wobei die hohlen Bereiche den Fluss des gepressten Materials während der Herstellung der Karte verbessern und das Bilden von "knit lines" bzw. Risslinien (81) in dem Material verhindern, so dass die Wahrscheinlichkeit der Kontamination zwischen unterschiedlichen Wannen in der Karte reduziert wird.

2. Karte nach Anspruch 1, wobei die ausgehöhlten Bereiche wenigstens in der ersten und zweiten Endregion der Karte angeordnet sind, die Wannen in einer Vielzahl von parallelen Reihen angeordnet sind, die eine erste Reihe benachbart zu der ersten Endregion und eine letzte Reihe benachbart zu der zweiten Endregion und die ausgehöhlten Bereiche aufweisen: Einen ersten Satz von ausgehöhlten Taschen, welche über den ersten Endbereich im Wesentlichen parallel und benachbart zu der ersten Reihe angeordnet sind, und einen zweiten Satz von ausgehöhlten Taschen, welche im Wesentlichen parallel und benachbart zu der letzten Reihe angeordnet sind, wobei vorzugsweise die ersten und zweiten ausgehöhlten Taschen langgestreckte ovale Aushöhltaschen aufweisen, welche eine Hauptachse definieren, welche so angeordnet ist, dass die Hauptachse senkrecht zu den ersten und letzten Reihen ausgerichtet ist.

3. Karte nach Anspruch 1 oder 2, wobei die ausgehöhlten Bereiche in wenigstens einer der ersten und zweiten Seitenbereiche angeordnet sind und die ausgehöhlten Bereiche wenigstens einen Dammbereich (35) aufweisen, welcher an der Grenzlinie des ausgehöhlten Bereiches und des nicht ausgehöhlten Teils der Karte angeordnet ist.

4. Karte nach einem der Ansprüche 1 bis 3, wobei die Karte eine obere Oberfläche (14) und einen Identifikationsbereich (50) in der oberen Oberfläche und eine untere Oberfläche (12) und einen ausgehöhlten Bereich in der unteren Oberfläche unter dem Identifikationsbereich aufweist, wobei vorzugsweise die Karte aus der Injektion gepressten Kristallpolystyrens hergestellt ist.

5. Karte nach einem der Ansprüche 1 bis 4, wobei der ausgehöhlte Bereich eine Tiefe zwischen 0,051 und 0,127 cm (zwischen 0,02 und 0,05 inch) besitzt, vorzugsweise im wesentlichen 0,076 cm (0,03 inch).

6. Karte nach Anspruch 1, welche einen Grundkörper aufweist, welcher aufweist: Einen Flüssigkeitseingangsanschluss und erste und zweite Endbereiche und erste und zweite Seitenbereiche, wobei der Grundkörper eine Vielzahl von Wannen definiert, welche zwischen den ersten und zweiten Endbereichen und den ersten und zweiten Seitenbereichen angeordnet sind, wobei der Grundkörper ferner aufweist: Ein Flüssigkeitsdurchlaufnetzwerk, welches den Flüssigkeitseingangsanschluss mit den Wannen verbindet, **dadurch gekennzeichnet, dass** wenigstens eine der Wannen eine Blasenfalle (28) besitzt, welche in Verbindung mit der Wanne über einen Blasenfallendurchlass (70) steht, wobei der Blasenfallendurchlass ein Wannenende und ein Blasenfallenende besitzt, wobei die Breite des Wannenendes größer als die Breite des Blasenfallenendes ist und der Schnittpunkt des Blasenfallenendes des Blasenfallendurchlasses und der Blasenfalle eine Einengung (74) in dem Blasenfallendurchlass bildet, wobei der Blasenfallendurchlass das Sammeln von Luftblasen erleichtert, welche in den Wannen in der Blasenfalle auftreten können, und die Einengung die Luftblasen am Durchtreten von der Blasenfalle in die Wanne hindert.

7. Karte nach Anspruch 6, wobei die Wanne ein erstes Endteil und ein gegenüberliegendes zweites Endteil aufweist, wobei die Blasenfalle benachbart zu dem ersten Endteil und einem Flüssigkeitseingangsdurchlauf, welcher in die Wanne an dem zweiten Endbereich eintritt, platziert ist.

8. Karte nach Anspruch 6 oder 7, wobei die Einengung ein scharfes Eck an der Kreuzung des Blasenfallendurchlasses und der Blasenfalle aufweist, wobei das scharfe Eck Luftblasen daran hindert, von der Blasenfalle in den Blasenfallendurchlass und in die Wanne sich zu bewegen.

9. Karte nach einem der Ansprüche 6 bis 8, wobei das Flüssigkeitsdurchlaufnetzwerk den Flüssigkeitseingangsanschluss mit der Wanne durch Flüssigkeitskanale verbindet, welche an den vorderen und rückwärtigen Oberflächen der Karte angeordnet sind, um dadurch den Trennungsabstand zwischen benachbarten Wannen zu erhöhen.

10. Karte nach Anspruch 1, wobei diese einen Grundkörper aufweist, welcher einen Flüssigkeitseingangsanschluss und erste und zweite Endbereiche und erste und zweite Seitenbereiche aufweist, wobei der Grundkörper eine Vielzahl von Wachstumswannen aufweist, welche zwischen den ersten und zweiten Endbereichen und den ersten und zweiten Seitenbereichen platziert sind, wobei der Grundkörper ferner eine obere Oberfläche und eine untere Oberfläche und ein Flüssigkeitsdurchlaufnetzwerk aufweist, welches den Flüssigkeitseingangsanschluss mit den Wachstumswannen verbindet, **dadurch gekennzeichnet, dass** das Flüssigkeitsdurchlaufnetzwerk einen ersten Satz von Flüssigkeitskanälen aufweist, welche in der oberen Oberfläche der Karte angeordnet sind, und einen zweiten Satz von Flüssigkeitskanälen aufweist, welche in der unteren Oberfläche der Karte angeordnet sind, wobei die ersten und zweiten Sätze der Flüssigkeitskanäle auf der Karte so angeordnet sind, dass sie einen adäquaten Trennungsabstand zwischen benachbarten Wannen liefern, wie dies entlang der Flüssigkeitskanäle gemessen wird, wodurch das Risiko der Kontamination zwischen benachbarten Wannen reduziert wird.

11. Karte nach Anspruch 10, wobei die Trennungsentfernung größer als 1,9 cm (0,75 inch) ist, vorzugsweise größer als 2,5 cm (1 inch).

12. Karte nach Anspruch 10 oder 11, wobei die Wannen in einer Serie von Reihen in der Karte mit fünf Wannen pro Reihe angeordnet sind und die Flüssigkeitskanäle zwischen dem Anschluss und den fünf Wannen der Reihe in der oberen und unteren Oberfläche der Karte so angeordnet sind, dass zwei Flüssigkeitskanäle in einer der Oberflächen der Karte angeordnet sind, welche zu zwei der Wannen in der Reihe von Wannen führen und drei Flüssigkeitskanäle auf der anderen der Oberflächen zu den verbleibenden drei der Wannen in der Reihe von Wannen angeordnet sind.

13. Karte nach Anspruch 1, wobei diese aufweist: Einen Grundkörper, welcher eine obere Oberfläche und eine untere Oberfläche besitzt, wobei Klebeband an den oberen und unteren Oberflächen angewendet wird, einen Flüssigkeitseingangsanschluss und erste und zweite Endbereiche und erste und zweite Seitenbereiche, wobei der Grundkörper eine Vielzahl von Wannen definiert, welche zwischen den ersten und zweiten Endbereichen und den ersten und zweiten Seitenbereichen platziert sind, wobei der Grundkörper ferner ein Flüssigkeitsdurchlaufnetzwerk aufweist, welches den Flüssigkeitseingangsanschluss mit den Wannen verbindet, **dadurch gekennzeichnet, dass** dieses ein Paar von Schienen (60) aufweist, welches sich aufwärts von einer der oberen oder unteren Oberflächen erstreckt, wobei die Schienen gestatten, dass die Karten aufeinander geschichtet werden können, ohne das Klebeband zu berühren, welches an benachbarten Karten in dem Stapel von Karten angewendet wird.

14. Karte nach Anspruch 13, wobei die Schienen entlang den ersten und zweiten Seitenbereichen zwischen den Enden der Karte angeordnet sind.

15. Karte nach Anspruch 6, wobei die Verengung in dem Blasenfallendurchlass eine Breite W1 von weniger als 0,1 cm (0,04 inch) besitzt.

16. Eine Form zum Herstellen einer Probenanalysekarte aus einem Kartenmaterial, **dadurch gekennzeichnet, dass** sie eine Vielzahl von Analysewannen aufweist, wobei die Karte eine äußere Oberfläche nach dem Auswerfen der Karte aus der Form besitzt, wobei die Form wenigstens ein positives Element besitzt, welches wenigstens einen ausgehöhlten Abschnitt in der Karte benachbart zu den Wannen erzeugt, wobei das positive Element den Fluss des Kartenmaterials in der Form so führt, um das Bilden von Risslinien in der Oberfläche zu steuern, um im Wesentlichen zu verhindern, dass sich die Risslinien in eine vorher festgelegte Richtung ausbilden.

17. Form nach Anspruch 16, wobei die Wannen in einer Vielzahl von Reihen angeordnet sind, welche eine Reihenrichtung definieren, und das positive Element ein langgestrecktes Element aufweist, welches benachbart zu den Reihen positioniert ist und welches eine lange Achse besitzt, welche senkrecht zu der Reihenrichtung ausgerichtet ist, wobei vorzugsweise das positive Element eine Dammeigenschaft aufweist, welches benachbart zu den Wannen platziert ist.

## Revendications

1. Carte (10) pour analyses d'échantillons comprenant un corps fabriqué à partir d'une matière fondue, ledit corps comprenant un orifice d'admission (16) de fluide, des première et deuxième régions d'extrémité (13, 15) et des première et deuxième régions latérales, ledit corps définissant une pluralité de puits (26) situés entre lesdites première et deuxième régions d'extrémité et lesdites première et deuxième régions latérales, ledit corps comprenant en outre un réseau de circulation de fluide (20) reliant ledit orifice d'admission de fluide auxdits puits, **caractérisée en ce que** ledit corps possède des régions creuses (32, 34, 54, 60) disposées dans au moins l'une desdites première et deuxième régions d'extrémité ou desdites première et deuxième régions latérales, les régions creuses améliorant l'écoulement de ladite matière fondue lors de la fabrication de ladite carte et empêchant la formation de lignes de soudure (81) dans ladite matière de façon à réduire les risques de contamination entre les différents puits de ladite carte.

2. Carte selon la revendication 1, dans laquelle lesdites régions creuses sont disposées dans au moins une desdites première et deuxième régions d'extrémité de ladite carte, lesdits puits sont agencés en une pluralité de rangées parallèles avec une première rangée adjacente à ladite première région d'extrémité et une dernière rangée adjacente à ladite deuxième région d'extrémité, et lesdites régions creuses comprennent un premier ensemble de poches creuses orientées en travers de ladite première région d'extrémité sensiblement de manière adjacente et parallèlement à ladite première rangée et un deuxième ensemble de poches creuses orientées sensiblement de manière adjacente et parallèlement à ladite dernière rangée, lesdites première et deuxième poches creuses comprenant de préférence des poches de carottage allongées et ovales définissant un axe principal agencé de façon à ce que l'axe principal soit orienté perpendiculairement auxdites première et dernière rangées.

3. Carte selon la revendication 1 ou la revendication 2, dans laquelle lesdites régions creuses sont disposées dans au moins une desdites première et deuxième régions latérales et lesdites régions creuses comprennent au moins une partie de retenue (35) disposée à la limite de ladite région creuse et de la partie non creuse de ladite carte.

4. Carte selon l'une quelconque des revendications 1 à 3, dans laquelle ladite carte possède une surface supérieure (14) et une région d'identification (50) dans ladite surface supérieure, ainsi qu'une surface inférieure (12) et une région creuse dans ladite surface inférieure sous ladite région d'identification, ladite carte étant de préférence obtenue à partir de polystyrène cristal moulé par injection.

5. Carte selon l'une quelconque des revendications 1 à 4, dans laquelle ladite région creuse a une profondeur comprise entre 0,051 et 0,127 cm (entre 0,02 et 0,05 pouce), de préférence sensiblement 0,076 cm (0,03 pouce).

6. Carte selon la revendication 1, comprenant un corps comprenant un orifice d'admission du fluide, des première et deuxième régions d'extrémité et des première et deuxième régions latérales, ledit corps définissant une pluralité de puits situés entre lesdites première et deuxième régions d'extrémité et lesdites première et deuxième régions latérales, ledit corps comprenant en outre un réseau de circulation de fluide reliant l'orifice d'admission de fluide auxdits puits, **caractérisée en ce qu'**au moins un desdits puits possède un piège à bulles (28) relié audit puits via un passage (70) de piège à bulles, ledit passage de piège à bulles possédant une extrémité côté puits et une extrémité côté piège à bulles, la largeur de ladite extrémité côté puits étant supérieure à la largeur de ladite extrémité côté piège à bulles, et l'intersection de ladite extrémité côté piège à bulles dudit passage de piège à bulles et dudit piège à bulles formant une restriction (74) dans ledit passage de piège à bulles, ledit passage de piège à bulles facilitant la collecte de bulles d'air pouvant être présentes dans lesdits puits dans ledit piège à bulles et ladite restriction empêchant les bulles d'air de sortir dudit piège à bulles pour entrer dans ledit puits.

7. Carte selon la revendication 6, dans laquelle ledit puits comprend une première partie d'extrémité et une deuxième partie d'extrémité opposée, ledit piège à bulles étant adjacent à ladite première partie d'extrémité, et un passage d'admission de fluide entrant dans ledit puits à la deuxième partie d'extrémité.

8. Carte selon la revendication 6 ou 7, dans laquelle ladite restriction comprend un angle aigu à l'intersection dudit passage de piège à bulles et dudit piège à bulles, ledit angle aigu empêchant les bulles de sortir dudit piège à bulles pour pénétrer dans ledit passage de piège à bulles et dans ledit puits.

9. Carte selon l'une quelconque des revendications 6 à 8, dans laquelle ledit réseau de circulation de fluide relie ledit orifice d'admission de fluide audit puits par des conduits de fluide disposés sur les surfaces avant et arrière de ladite carte de façon à augmenter la distance de séparation entre les puits adjacents.

10. Carte selon la revendication 1, comprenant un corps comprenant un orifice d'admission de fluide, des première et deuxième régions d'extrémité et des première et deuxième régions latérales, ledit corps définissant une pluralité de puits de croissance situés entre lesdites première et deuxième régions d'extrémité et lesdites première et deuxième régions latérales, ledit corps comprenant en outre une surface supérieure et une surface inférieure ainsi qu'un réseau de circulation de fluide reliant ledit orifice d'admission de fluide auxdits puits de croissance, **caractérisée en ce que** le réseau de circulation de fluide comprend un premier ensemble de conduits de fluide disposés dans ladite surface supérieure de ladite carte, et un deuxième ensemble de conduits de fluide disposés dans ladite surface inférieure de ladite carte, lesdits premier et deuxième ensembles de conduits de fluide étant agencés sur ladite carte de façon à créer une distance de séparation appropriée entre les puits adjacents mesurés le long desdits conduits de fluide, réduisant ainsi le risque de contamination entre les puits adjacents.

11. Carte selon la revendication 10, dans laquelle ladite distance de séparation est supérieure à 1,9 cm (0,75 pouce), de préférence supérieure à 2,5 cm (1 pouce).

12. Carte selon la revendication 10 ou la revendication 11, dans laquelle lesdits puits sont agencés en une série de rangées dans ladite carte avec cinq puits par rangée, et lesdits conduits de fluide entre ledit orifice et lesdits cinq puits de ladite rangée sont disposés sur lesdites surfaces supérieure et inférieure de ladite carte de sorte que deux conduits de fluide soient disposés sur l'une desdites surfaces de ladite carte menant à deux desdits puits dans ladite rangée de puits et que trois conduits de fluide soient disposés sur l'autre desdites surfaces menant aux trois desdits puits restants dans ladite rangée de puits.

13. Carte selon la revendication 1 comprenant un corps possédant une surface supérieure et une surface inférieure, une bande adhésive étant appliquée sur lesdites surfaces supérieure et inférieure, un orifice d'admission de fluide, des première et deuxième régions d'extrémité et des première et deuxième régions latérales, ledit corps définissant une pluralité de puits situés entre lesdites première et deuxième régions d'extrémité et lesdites première et deuxième régions latérales, ledit corps comprenant en outre un réseau de circulation de fluide reliant ledit orifice d'admission de fluide auxdits puits, **caractérisée en ce qu'**elle comprend une paire de glissières (60) s'étendant vers le haut en s'éloignant d'une desdites surfaces supérieure ou inférieure, lesdites glissières permettant à ladite carte d'être empilée sans toucher la bande adhésive appliquée aux cartes adjacentes dans ladite pile de cartes.

14. Carte selon la revendication 13, dans laquelle lesdites glissières sont orientées le long desdites première et deuxième régions latérales entre lesdites extrémités de ladite carte.

15. Carte selon la revendication 6, dans laquelle ladite restriction dans ledit passage de piège à bulles a une largeur, Wl, inférieure à 0,1 cm (0,04 pouce).

16. Moule de fabrication d'une carte pour analyses d'échantillons à partir d'une matière de carte, **caractérisé en ce qu'**il comprend une pluralité de puits d'échantillons, ladite carte possédant une surface extérieure après l'éjection de ladite carte dudit moule, ledit moule ayant au moins un élément positif créant au moins une section creuse dans ladite carte adjacente auxdits puits, ledit élément positif dirigeant l'écoulement de ladite matière de carte dans ledit moule de façon à contrôler sensiblement la formation de lignes de soudure sur la surface de ladite carte afin d'empêcher lesdites lignes de soudure de se former dans une direction prédéterminée.

17. Moule selon la revendication 16, dans lequel lesdits puits sont agencés en une pluralité de rangées définissant une direction de rangée et ledit élément positif comprend un élément allongé adjacent auxdites rangées et possédant un long axe orienté perpendiculairement à ladite direction de rangée, ledit élément positif comprenant de préférence une retenue adjacente auxdits puits.
